# EUROPEAN PATENT APPLICATION

(11) **EP 1 792 977 A1**
(43) Date of publication of application: **06.06.2007**
(21) Application number: 05774577.0
(22) Date of filing: 23.08.2005
(51) Int. Cl.: C12N 5/00, A61K 35/32, A61L 27/00, A61P 19/00

(54) **MEDIUM FOR THE REDIFFERENTIATION OF DEDIFFERENTIATED CHONDROCYTES INTO CHONDROCYTES**

(30) Priority: 24.08.2004 JP 2004244114
(71) Applicant: Fujisoft Incorporated, Kanagawa 231-8008 (JP); Hoshi, Kazuto, Bunkyo-ku Tokyo 112-0012 (JP)
(72) Inventor: HOSHI, Kazuto, Tokyo 112-0012 (JP); TEI, Yuichi, The University of Tokyo,Faculty of Medicine, Tokyo 113-0033 (JP); KAWAGUCHI, Hiroshi, Tokyo 113-0022 (JP); NAKAMURA, Kozo, Tokyo 179-0081 (JP); TAKATO, Tsuyoshi, Tokyo 167-0052 (JP)
(74) Representative: Bassil, Nicholas Charles
(86) International application number: PCT/JP2005/015280
(87) International publication number: WO 2006/022263

(57) **Abstract**

It is intended to provide a medium for the redifferentiation of dedifferentiated chondrocytes into chondrocytes and a method of redifferentiating dedifferentiated chondrocytes into chondrocytes whereby dedifferentiated chondrocytes, which have been dedifferentiated by, for example, culturing *in vitro* and thus show weakened chondral characteristics, can be efficiently and easily redifferentiated into the original chondrocytes. That is to say, a medium for the redifferentiation of dedifferentiated chondrocytes into chondrocytes which is to be used for redifferentiating dedifferentiated chondrocytes having been dedifferentiated and thus showing weakened chondral characteristics into the original chondrocytes and contains insulin together with at least one member selected from among BMP-2 and its analogs; and a method of redifferentiating dedifferentiated chondrocytes into the original chondrocytes by culturing the dedifferentiated chondrocytes with the use of the medium for the redifferentiation of dedifferentiated chondrocytes into chondrocytes. In a preferred embodiment, the above-described medium for the redifferentiation of dedifferentiated chondrocytes into chondrocytes further contains T3.

## Description

### Technical Field

The present invention relates to a redifferentiation medium used for making dedifferentiated chondrocyte to be redifferentiated into original chondrocyte, cartilage characteristics of said dedifferentiated chondrocyte having been attenuated due to dedifferentiation during in vitro culture.

### Background Art

Cartilage constitutes ear, nose, trachea, joint and intervertebral disc, and is as important as bones for maintaining physical morphology of human and for effecting daily activity of life. When the cartilage is impaired by trauma such as damage of articular cartilage, aging-related diseases such as arthrosis deformans, inflammatory diseases such as rheumatoid arthritis, large-sized cartilage defect after surgery of tumors, and congenital anomaly, daily life is severely impaired such that life activities such as walking become difficult and normal posture cannot be maintained. The number of patients affected with these cartilage-related diseases is very large, and the number of the patients manifesting the arthrosis deformans is estimated to be about 900,000 per year. Therefore, effective therapy for these diseases is desired.

While these diseases have been conventionally treated by using artificial cartilage or by transplantation of patient's own cartilage, these methods often cause problems of durability, infection and donor site troubles. Therefore, solving means without these problems has been desired and development of technology for enabling regenerative therapy of the cartilage is urgently expected.

Technology for efficiently culturing the chondrocyte is essential for regenerative therapy of the cartilage. While many methods for culturing and proliferating the chondrocyte have been reported (see patent documents 1 and 2), the chondrocyte is dedifferentiated to fibroblast-like cells during culture with a quite high probability. As countermeasures and solving means for such a case the chondrocyte has been changed to fibloblast-like dedifferentiated chondrocyte, there are proposed a method for redifferentiating the dedifferentiated chondrocyte using a hydrostatic pressure, and a method for redifferentiating the dedifferentiated chondrocyte by three-dimensional culture. However, it is the status quo that the dedifferentiated chondrocyte cannot be easily and efficiently redifferentiated into the original chondrocyte by these methods. In order to realize the cartilage-regenerating treatment, therefore, it is desired to provide technology capable of efficiently and readily redifferentiating the dedifferentiated chondrocyte into original chondrocyte, cartilage characteristics of said dedifferentiated chondrocyte having been attenuated due to dedifferentiation during in vitro culture.

Patent document 1: Jpn. Pat. Appln. KOKAI Publication No. 2003-534792

Patent document 2: Jpn. Pat. Appln. KOKAI Publication No. 2004-502401

### Disclosure of Invention

The present invention is directed to solving the conventional problems and attaining the following object.

Namely, the object of the invention is to provide a medium for making dedifferentiated chondrocyte to be redifferentiated into chondrocyte and a method for making dedifferentiated chondrocyte to be redifferentiated into chondrocyte, the medium and the method being important for efficiently producing transplantation materials for deficient or impaired cites of nose, ear, trachea and joint and cosmetic materials for cosmetic surgery; being able to contribute to the development of regenerative medicine of the cartilage; and being able to efficiently and readily redifferentiate the dedifferentiated chondrocyte, which has been changed to fibroblast-like cells by dedifferentiation during in vitro culture, into the original chondrocyte.

As a result of intensive studies by the inventors of the invention by taking the status quo into consideration, it has been found that the dedifferentiated chondrocyte, in which characteristics of the cartilage have been attenuated by dedifferentiation, can be efficiently and readily redifferentiated into the original chondrocyte by using combination of insulin and BMP-2, and preferably together with T3. The invention is based on the above-mentioned discovery by the inventors, and means for solving the above-mentioned problems are as follows:
<1> a redifferentiation medium used for making dedifferentiated chondrocyte to be redifferentiated into original chondrocyte, cartilage characteristics of said dedifferentiated chondrocyte having been attenuated due to dedifferentiation during in vitro culture, said medium comprising: insulin; and at least one member selected from the group consisting of BMP-2 and analogues thereof;
<2> the redifferentiation medium for making dedifferentiated chondrocyte to be redifferentiated into chondrocyte according to <1>, further comprising T3;
<3> the redifferentiation medium for making dedifferentiated chondrocyte to be redifferentiated into chondrocyte according to any one of <1> and <2>, wherein the analogue of BMP-2 is BMP-4;
<4> the redifferentiation medium for making dedifferentiated chondrocyte to be redifferentiated into chondrocyte according to any one of <1> to <3>, wherein the insulin is present at a concentration from 0.05 to 500 µg/mL;
<5> the redifferentiation medium for making dedifferentiated chondrocyte to be redifferentiated into chondrocyte according to any one of <1> to <4>, wherein the at least one member selected form the group consisting of BMP-2 and analogues thereof is present at a concentration from 1 ng/mL to 40 µg/mL;
<6> the redifferentiation medium for making dedifferentiated chondrocyte to be redifferentiated into chondrocyte according to any one of <1> to <5>, wherein T3 is present at a concentration from 10-9 to 10-5 M;
<7> the redifferentiation medium for making dedifferentiated chondrocyte to be redifferentiated into chondrocyte according to any one of <1> to <6>, further comprising at least one member selected from the group consisting of fibroblast growth factor 2 (FGF-2), insulin-like growth factors (IGF-1), parathyroid hormone (PTH), growth hormone (GH), glucocorticoid, vitamin D, IL-1 receptor antagonist, estrogen, androgen, transformation growth factor α (TGFα), transformation growth factor β (TGFβ), bone morphogenic proteins (BMP), epidermal growth factor, platelet-derived growth factors, transferrin, selenious acid, linoleic acid, albumin, ascorbic acid, chondromodulins, heparin binding factor, α-fibroblast growth factor, vascular endothelial growth factor, mitogenic hormone, connective tissue growth factor, hepatocyte growth factor, arachidonic acid, prostaglandin A, prostaglandin B, prostaglandin E, prostaglandin F and histamine;
<8> the redifferentiation medium for making dedifferentiated chondrocyte to be redifferentiated into chondrocyte according to any one of <1> to <7>, wherein the redifferentiated chondrocyte is selected from hyaline chondrocyte and elastic chondrocyte.
<9> the redifferentiation medium for making dedifferentiated chondrocyte to be redifferentiated into chondrocyte according to any one of <1> to <8>, wherein the redifferentiated chondrocyte is cultured by any one of plane culture, three-dimensional culture and pellet culture;
<10> a redifferentiation method for making dedifferentiated chondrocyte to be redifferentiated into original chondrocyte, cartilage characteristics of said dedifferentiated chondrocyte having been attenuated due to dedifferentiation during in vitro culture, said method comprising the step of redifferentiating the dedifferentiated chondrocyte into the original chondrocyte by culturing the dedifferentiated chondrocyte using the medium according to any one of <1> to <9>;
<11> the redifferentiation method according to <10>, wherein culturing is continued for 3 to 6 weeks;
<12> the redifferentiation method according to any one of <10> and <11>, wherein the ratio (C/D) of the amount (C) of expression and production of type II collagen in the redifferentiated chondrocyte to the amount (D) of expression and production of type II collagen in the dedifferentiated chondrocyte is 1 or more;
<13> the redifferentiation method according to any one of <10> to <12>, wherein the expression/production amount of type I collagen in the dedifferentiated chondrocyte is equal to or more than the expression/production amount of type I collagen in the chondrocyte in the living body, the amount of production of type II collagen is equal to or less than the expression/production amount of type II collagen in the chondrocyte in the living body, and the expression/production amount of type II collagen is equal to or more than the expression/production amount of type II collagen in the dedifferentiated chondrocyte; and
<14> the redifferentiation method according to any one <10> to <13>, wherein compression strength, fracture strength and Young's modulus in the dedifferentiated chondrocyte are larger than those in the redifferentiated chondrocyte.

### Brief Description of Drawings

FIG. 1 shows photographs, in which the left one shows the chondrocyte (P0) immediately after starting culture, and the right one shows the chondrocyte (P4) at the fourth passaged subculture about 30 days after the start of culture, respectively. The photographs at the top show electrophoresis bands showing expression of genes in the chondrocyte (P0) immediately after starting culture and in the chondrocyte (P4) at the fourth passaged subculture about 30 days after the start of culture.
FIG. 2 is a graph showing the results of measurement of the expression amount of type I collagen in the dedifferentiated chondrocyte cultured using a redifferentiation medium containing insulin, BMP-2 and T3, and using a culture medium containing insulin and BMP-2.
FIG. 3 is a graph showing the results of measurement of the expression amount of type II collagen in the dedifferentiated chondrocyte cultured using a redifferentiation medium containing insulin, BMP-2 and T3, and using a culture medium containing insulin and BMP-2.
FIG. 4 is a graph showing the results of measurement of the expression amount of type X collagen in the dedifferentiated chondrocyte cultured using a redifferentiation medium containing insulin, BMP-2 and T3, and using a culture medium containing insulin and BMP-2.
FIG. 5 is a graph showing data obtained by studying and comparing preferable ranges of the content of BMP-2 in the redifferentiation medium containing insulin, BMP-2 and T3 for redifferentiating the dedifferentiated chondrocyte into the chondrocyte in Example 2.
FIG. 6 is a graph showing data obtained by studying and comparing preferable ranges of the content of insulin in the redifferentiation medium containing insulin, BMP-2 and T3 for redifferentiating the dedifferentiated chondrocyte into the chondrocyte in Example 2.
FIG. 7 is a graph showing data obtained by studying and comparing preferable ranges of the content of T3 in the redifferentiation medium containing insulin, BMP-2 and T3 for redifferentiating the dedifferentiated chondrocyte into the chondrocyte in Example 2.
FIG. 8 is a graph showing the results of measurement of the expression amount of type I collagen in the redifferentiation medium for redifferentiating the cultured dedifferentiated chondrocyte when the contents of insulin, BMP-2 and T3 are 5 µg/mL, 200 ng/mL and 10-7 M, respectively, and in the redifferentiation medium having the same composition as described above except that BMP-2 is changed to BMP-4.
FIG. 9 is a graph showing the results of measurement of the expression amount of type II collagen in the redifferentiation medium for redifferentiating the cultured dedifferentiated chondrocyte when the contents of insulin, BMP-2 and T3 are 5 µg/mL, 200 ng/mL and 10-7 M, respectively, and in the redifferentiation medium having the same composition as described above except that BMP-2 is changed to BMP-4.
FIG. 10 is a graph showing the results of measurement of the expression amount of type X collagen in the redifferentiation medium for redifferentiating the cultured dedifferentiated chondrocyte when the contents of insulin, BMP-2 and T3 are 5 µg/mL, 200 ng/mL and 10-7 M, respectively, and in the redifferentiation medium having the same composition as described above except that BMP-2 is changed to BMP-4.
FIG. 11 is a graph showing the results of measurement of the compression strength as a mechanical property of three-dimensional culture products obtained by three-dimensional culture.
FIG. 12 shows photographic data of the three-dimensional culture products obtained by three-dimensional culture.
FIG. 13 shows a graph showing the results of measurement of the compression strength as a mechanical property of the culture product which was obtained by using the culture redifferentiation medium for redifferentiating the dedifferentiated chondrocyte into the chondrocyte, and subcutaneously transplanted into a nude mouse for 2 months.
FIG. 14 shows photographic data of the culture product which was obtained by using the culture redifferentiation medium for redifferentiating the dedifferentiated chondrocyte into the chondrocyte, and subcutaneously transplanted into a nude mouse for 2 months.

### Best Mode for Carrying Out the Invention

(redifferentiation medium for making dedifferentiated chondrocyte to be redifferentiated into chondrocyte, and method for making dedifferentiated chondrocyte to be redifferentiated into chondrocyte)

The redifferentiation medium according to the present invention for redifferentiating the dedifferentiated chondrocyte into the chondrocyte is used for redifferentiating the dedifferentiated chondrocyte into original chondrocyte, cartilage characteristics of said dedifferentiated chondrocyte having been attenuated due to dedifferentiation during in vitro culture. The redifferentiation medium comprises insulin and at least one of BMP-2 and analogues thereof, and preferably, further comprises T3, and optionally, further comprises suitably selected other components.

The redifferentiation method according to the present invention for redifferentiating the dedifferentiated chondrocyte into the chondrocyte comprises the step of redifferentiating the dedifferentiated chondrocyte into original chondrocyte, by culturing the dedifferentiated chondrocyte in the redifferentiation medium of the present invention, and optionally, an aditional step of other treatment appropriately selected, cartilage characteristics of said dedifferentiated chondrocyte having been attenuated due to dedifferentiation during in vitro culture,

The redifferentiation medium of the invention for redifferentiating the dedifferentiated chondrocyte to chondrocyte, and the method of the invention for redifferentiating the dedifferentiated chondrocyte to the chondrocyte will be described below.

Insulin used is not particularly restricted, and may be appropriately selected depending on the purpose. For example, it may be either commercially available insulin or appropriately synthesized insulin.

While the content of insulin in the redifferentiation medium for redifferentiating the dedifferentiated chondrocyte into the chondrocyte is not particularly restricted and may be appropriately selected depending on the purpose, the content is preferably from 0.05 to 500 µg/mL.

Redifferentiation may not be always induced when the content of insulin is less than 0.05 µg/mL, while induction of redifferentiation may be inhibited when the content exceeds 500 µg/mL.

Above-mentioned BMP-2 denotes bone morphogenic protein-2. The BMP-2 is not particularly restricted and may be appropriately selected depending on the purpose, and it may be either a commercially available product or an appropriately synthesized product.

While analogue of BMP-2 is not particularly restricted and may be appropriately selected from those known in the art, a favorable example is BMP-4.

As for at least one selected from BMP-2 and analogues thereof, use can be made of BMP-2 and BMP-4 in combination. In addition, while BMP-2 or BMP-4 may be used alone, BMP-2 is preferably used alone.

In the redifferentiation medium for redifferentiating the dedifferentiated chondrocyte into the chondrocyte, the content of at least one selected from BMP-2 and analogies thereof is not particularly restricted, and may be appropriately selected depending on the purpose, but the preferable content is, for example, from 1 ng/mL to 40 µg/mL.

Redifferentiation may not be induced when the content of at least one selected from BMP-2 and analogies thereof is less than 1 ng/mL, while induction of redifferentiation may be inhibited when the content exceeds 40 µg/mL.

T3 denotes thyroid hormone (triiodothryonine). While T3 is not particularly restricted and may be appropriately selected depending on the purpose; for example, either a commercially available product or an appropriately synthesized product may be used.

The redifferentiation medium for redifferentiating the dedifferentiated chondrocyte into the chondrocyte further containing T3 is advantageous over the medium containing insulin and at least one selected from BMP-2 and analogues thereof without T3, since the dedifferentiated chondrocyte can be more efficiently redifferentiated while expression of type X collagen that increases during osteogenesis can be efficiently suppressed.

While the content of T3 in the redifferentiation medium for redifferentiating the dedifferentiated chondrocyte into the chondrocyte is not particularly restricted and may be appropriately selected depending on the purpose, it is preferably from 10-9 to 10-5 M.

Redifferentiation may not be efficiently induced when the content of T3 is less than 10-9 M, while redifferentiation may be inhibited when the content exceeds 10-5 M. When the content of T3 is within the above-mentioned range, the dedifferentiated chondrocyte can be more efficiently redifferentiated as compared with the medium containing insulin and at least one selected from BMP-2 and analogues thereof without T3, while type X collagen may be efficiently suppressed from being expressed.

While other components are not particularly restricted and may be appropriately selected depending on the purpose, examples thereof include components that may affect redifferentiation of the dedifferentiated chondrocyte into the chondrocyte and solvents.

The components that may affect redifferentiation of the dedifferentiated chondrocyte into the chondrocyte are not particularly restricted and may be appropriately selected depending on the purpose. Examples of the component include fibroblast growth factor 2 (FGF-2), insulin-like growth factors (IGF-1), parathyroid hormone (PTH), growth hormone (GH), glucocorticoid (dexamethasone), vitamin D, IL-1 receptor antagonist, estrogen, androgen (such as testosterone), transformation growth factor α (TGFα), transformation growth factor β (TGFβ), bone morphogenic proteins (BMP), epidermal growth factor, platelet-derived growth factors, transferrin, selenious acid, linoleic acid, albumin, ascorbic acid, chondromodulins, heparin binding factor, α-fibroblast growth factor, intravascular growth factor, cell division accelerating hormone, connective tissue growth factor, hepatocyte growth factor, arachidonic acid, prostaglandin A, prostaglandin B, prostaglandin E, prostaglandin F and histamine.

Each of the other components described above may be used alone, or a plurality of them may be used in combination.

The solvent is not particularly restricted and may be appropriately selected depending on the purpose. A favorable example is water.

Water includes sterilized water and Millipore Q water.

The content (total content) of the other components in the redifferentiation medium for redifferentiating the dedifferentiated chondrocyte into the chondrocyte is not particularly restricted, and may be appropriately selected depending on the purpose.

The dedifferentiated chondrocyte as the target of the redifferentiation medium of the invention for redifferentiating the dedifferentiated chondrocyte into the chondrocyte is a cell derived from the chondrocyte, cartilage characteristics of which have been attenuated by dedifferentiation during in vitro culture.

The cartilage characteristics as used herein means the expression/production amount of type I collagen and type II collagen in the living body. Attenuation of the cartilage characteristics means that the ratio (A/B) of the amount (A) of expression and production of type I collagen to the amount (B) of expression and production of type I collagen in the chondrocyte (pre-dedifferentiated chondrocyte) before the chondrocyte is dedifferentiated is 1 or more, or that the ratio (C/D) of the amount (C) of expression and production of type II collagen to the amount (D) of expression and production of type II collagen in the chondrocyte (pre-dedifferentiated chondrocyte) before the chondrocyte is dedifferentiated is 1 or less.

The pre-dedifferentiated chondrocyte means the chondrocyte in the living body from which the dedifferentiated chondrocyte is derived.

Whether the chondrocyte is the dedifferentiated chondrocyte having attenuated cartilage characteristics, the pre-dedifferentiated chondrocyte or the chondrocyte (redifferentiated chondrocyte) redifferentiated from the dedifferentiated chondrocyte may be judged from the expression/production amount of type I collagen, the expression/production amount of type II collagen, and the expression/production amount of type X collagen, the compression strength, fracture strength, Young's modulus and equilibrium compression coefficient.

The cell in which the expression/production amount of type II collagen is smaller than in the chondrocyte in the living body, the expression/production amount of type I collagen is larger than in the chondrocyte in the living body, and the compression strength, fracture strength, Young's modulus and equilibrium compression coefficient are lower than in the chondrocyte in the living body may be judged to be highly possible to be the dedifferentiated chondrocyte, while the cell in which the expression/production amount of type I collagen is smaller than in the chondrocyte in the living body, the expression/production amount of type II collagen is larger than in the chondrocyte in the living body, and the compression strength, fracture strength, Young's modulus and equilibrium compression coefficient are higher than in the chondrocyte in the living body may be judged to be highly possible to be the redifferentiated chondrocyte.

Since the chondrocyte produces a cartilage matrix and is metachromatic to toluidine blue, the non-dedifferentiated or redifferentiated chondrocyte can be discriminated by toluidine blue staining.

In the chondrocyte, generally, the expression/production amount of type I collagen is small, the expression/production amount of type X collagen that increases when converted into the bone is small, the expression/production amount of type II collagen is large, and expression of at least one of COL2A1 gene, COL9A1 gene, COL11A2 gene, Aggrecan gene, Matrillin 3 gene and Chondromodulin 1 gene is higher than in the dedifferentiated chondrocyte.

Accordingly, the chondrocyte is likely to be changed to fibroblast-like dedifferentiated chondrocyte when type I collagen is expressed and produced in the chondrocyte. Since COL2A1 gene, COL9A1 gene, COL11A2 gene, Aggrecan gene, Matrillin 3 gene and Chondromodulin 1 gene are highly expressed in the normal chondrocyte, these genes and proteins such as type II collagen as the results of expression of these genes may be markers of the normal chondrocyte. Proteoglycan may also serve as the marker of the normal chondrocyte in addition to these proteins.

Specific examples of the chondrocyte include hyaline chondrocyte such as articular chondrocyte (chondrocyte derived from non-loaded portion of the articular cartilage) and costal chondrocyte (chondrocyte derived from costal cartilage); and elastic chondrocyte such as auricular chondrocyte (chondrocyte derived from auricular cartilage).

While each of these chondrocytes is usually used alone, a plurality of them may be used together.

Examples of the redifferentiated chondrocyte are the same as the pre-dedifferentiated chondrocyte. While the redifferentiated chondrocyte is not particularly restricted as long as the ratio (C/D) of the expression amount of the type II collagen (C) to the expression amount of the type II collagen (D) in the dedifferentiated chondrocyte exceeds 1, the ratio is preferably 10 or more, more preferably from 100 to 1000.

While the amount of seeding of the dedifferentiated chondrocyte on the medium for redifferentiating the dedifferentiated chondrocyte into the chondrocyte is not particularly restricted and may be appropriately selected depending on the purpose, it is preferably from about 105 to about 109 cells/mL, more preferably from about 106 to about 108 cells/mL.

The cell may be hypertrophic when the amount of seeding of the dedifferentiated chondrocyte is less than 105 cells/mL, while the cell may be hypoxic and hypotrophic when the amount of seeding of the dedifferentiated chondrocyte exceeds 109 cells/mL.

The dedifferentiated chondrocyte seeded on the medium for redifferentiating the dedifferentiated chondrocyte into the chondrocyte is not particularly restricted, and may be appropriately selected depending on the purpose. While favorable examples of the dedifferentiated chondrocyte include those changed to the dedifferentiated chondrocyte by dedifferentiation of the chondrocyte during in vitro culture, and those extracted as the dedifferentiated chondrocyte, the former is more preferable.

The culture condition of the dedifferentiated chondrocyte in the redifferentiation medium for redifferentiating the dedifferentiated chondrocyte into the chondrocyte is not particularly restricted, and may be appropriately selected depending on the purpose.

While the culture time is not particularly restricted and may be appropriately selected depending on the purpose, it is usually 1 week or more, preferably from 3 to 6 weeks.

Redifferentiation may be insufficient when the culture time is less than 1 week.

While the culture temperature is not particularly restricted and may be appropriately selected depending on the purpose, it is usually from 32 to 42°C, preferably from 34 to 39°C.

Redifferentiation may be impaired or the cell may die when the culture temperature is less than 32°C.

While the oxygen partial pressure in the culture is not particularly restricted and may be appropriately selected depending on the purpose, it is usually from 10 to 30%, preferably from 15 to 25%.

Redifferentiation may be impaired or the cell may die when the oxygen partial pressure is less than 10%.

While the pH in the culture is not particularly restricted and may be appropriately selected depending on the purpose, it is usually from 6 to 8, preferably from 6.5 to 7.5.

Redifferentiation may be impaired or the cell may die when the pH is less than 6 or exceeds 8.

The culturing method is not particularly restricted and may be appropriately selected depending on the purpose. Examples of the culturing method include plate culture (monolayer culture), three-dimensional culture and pellet culture.

Each of these methods may be used alone, or a plurality of the methods may be used in combination. The three-dimensional culture is preferable among them.

The plate culture (monolayer culture) is two-dimensional culture effected on a plate or Petri dish.

In the three-dimensional culture, the cells are cultured on or within a three-dimensional matrix (scaffold material) made of collagen, fibrin or hyaluronic acid.

Commercially available materials such as aterocollagen (manufactured by Kawaken Fine Chemicals Co.) may be used for the three-dimensional culture.

The three-dimensional matrix (scaffold material) is not particularly restricted, and the material, property, shape, structure and size thereof may be appropriately selected depending on the purpose.

Examples of the scaffold material include poly-D-lactide, poly-L-lactide, poly-DL-lactide, polyglycolic acid, polylactic acid, hyroxyapatite, calcium phosphate, calcium phosphate, hyroxyapatite, aterocollagen, collagen, fibrin, alginate, agar and gelatin. One of these materials may be used alone, or a plurality of the material may be used together. Aterocollagen is favorable among them.

A favorable property of the matrix is a gel.

The shape may be appropriately selected depending on the required shape for the materials used for transplantation or cosmetic materials.

Examples of the favorable structure generally include porous structure, mesh structure and sponge structure.

In the pellet culture, the chondrocyte cultured by the plate culture is peeled, and is cultured in the culture medium with being gently centrifuged. Round pellets of the cultured cells are obtained by the pellet culture. By the pellet culture, it is possible to make resultant cultured cells to be coagulated in a high density.

According to the redefferentiation method of the present invention in which the dedifferentiated chondrocyte is redifferentiated into the condrocyte by using the redifferentiation medium of the present invention for redifferentiating the dedifferentiated chondrocyte into the chondrocyte, the resultant redifferentiated chondrocyte can be used to form transplantation materials for the impaired or damaged cartilage of the nose, ear, trachea and joint, and for cosmetic materials for cosmetic surgery.

The method for culturing or proliferating the resultant redifferentiated chondrocyte is not particularly restricted, and may be appropriately selected depending on the purpose. For example, the method includes above-mentioned plate culture (monolayer culture), three-dimensional culture and pellet culture.

While each of these methods may be used alone or a plurality of the methods may be used in combination, the three-dimensional culture method is favorable among others.

Although there is a problem that the chondrocyte is relatively easily dedifferentiated and changed to a fibroblast-like dedifferentiated chondrocyte when the chondrocyte is cultured and proliferated in vitro for the purpose of regenerative medicine of the cartilage, the dedifferentiated chondrocyte can be efficiently and readily redifferentiated into the chondrocyte by using the redifferentiation medium of the present invention for redifferentiating the dedifferentiated chondrocyte into the chondrocyte, or by using the method of the present invention for redifferentiating the dedifferentiated chondrocyte into the chondrocyte. Accordingly, the resultant chondrocyte (mass of the chondrocyte) obtained by further culturing the redifferentiated chondrocyte may be favorably used for treating various diseases related to the cartilage by embedding the chondrocyte into a cartilage deficient cite. The invention may be favorably applied to regenerative therapy of the cartilage, since the chondrocyte extracted from the patient himself can be cultured and returned to the patient in order to use the cell as the cartilage (mass of the chondrocyte).

### Examples

While examples of the invention will be described in detail below, the invention is not restricted any way to these examples.

### - Differentiation of chondrocyte into dedifferentiated chondrocyte due to dedifferentiation -

A chondrocyte proliferation medium (10 mL; manufactured by Cambrex Co.) containing FGF-2 (fibroblast growth factor-2), IGF-1 (insulin-like growth factor-1), insulin, transferrin and selenic acid was added to 5% by mass of FBS (fetal bovine serum) in a Petri dish, and the chondrocyte (derived from human auricular cartilage; 200,000 cells) was subcultured in the Petri dish for 30 days by plate culture (monolayer culture). The plate culture (monolayer culture) condition was 37°C, 30 days, pH 7 and oxygen partial pressure of 20%.

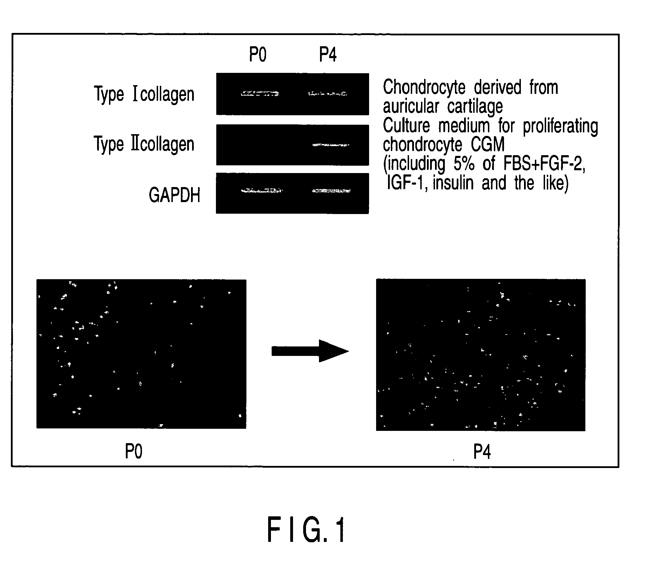

In FIG. 1, the photograph at the left shows chondrocyte (P0) immediately after starting culture, and the photograph at the right shows the chondrocyte (P4) at fourth subculture about 30 days after the start of culture. The photographs at the top of FIG. 1 show electrophoresis bands showing expression of genes of type I collagen, type II collagen and GADPH (glyceroaldehyde-6-phoaphate dehydrogenase) in the chondrocyte (P0) immediately after starting culture and in the chondrocyte (P4) at fourth subculture about 30 days after the start of culture.

These photographs show that type II collagen was expressed and type I collagen was not expressed in the chondrocyte (P0) immediately after the start of culture. This shows the characteristics of the chondrocyte (pre-dedifferentiated chondrocyte), and the chondrocyte (P0) immediately after the start of culture was confirmed not to be dedifferentiated. On the other hand, the expression amount of type II collagen was decreased while type I collagen was expressed in the chondrocyte (P4) at fourth subculture about 30 days after the start of culture. This shows the characteristics of the dedifferentiated chondrocyte, and it was confirmed that the chondrocyte (P4) at fourth subculture about 30 days after the start of culture was dedifferentiated.

### - Redifferentiation of dedifferentiated chondrocyte into chondrocyte -

The chondrocyte (P4: dedifferentiated chondrocyte, 200,000 cells) at fourth subculture about 30 days after the start of culture was cultured by embedding in a three-dimensional matrix of aterocollagen for 7 days using a medium (a medium in Example 1 for redifferentiating the dedifferentiated chondrocyte into the chondrocyte) containing 5 µg/mL of insulin (manufactured by MP Biochemicals Co.) and 200 nm/mL of BMP-2 (human recombinant bone morphogenetic protein-2, manufactured by Yamanouchi Pharmaceutical Co.) in a basal medium Dulbecco's Modified Eagle's Medium Nutrient Mixture F-12 HAM (trade name: DMEM/F12, manufactured by Sigma Chemical Co.), and using a medium (a medium in Example 2 for redifferentiating the dedifferentiated chondrocyte into the chondrocyte) containing 10-7 M of T3 (L-3,3',5'-triiodothyronine) in addition to the above-mentioned components in the basal medium. This culture corresponds to the method of the invention for redifferentiating the dedifferentiated chondrocyte into the chondrocyte.

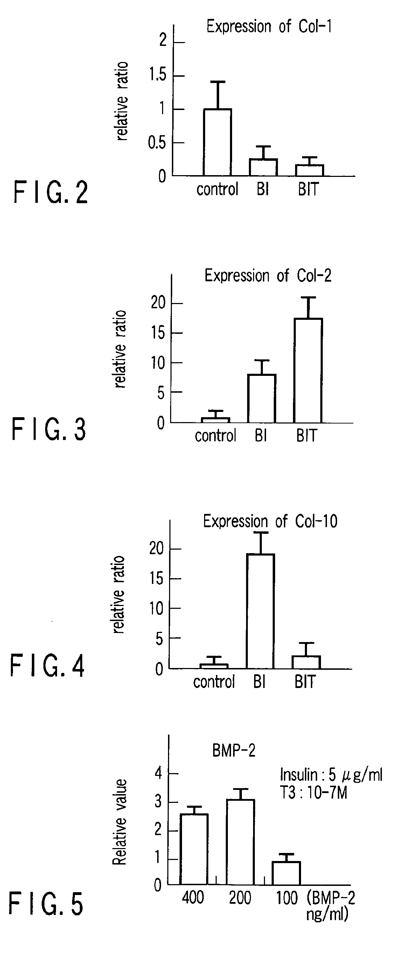

The amounts of expression of type I collagen (Col-I), type II collagen (Col-II) and type X collagen (Col-X) in the dedifferentiated chondrocytes cultured using the above-mentioned two culture media were measured. The amounts of expression of type I collagen, type II collagen and type X collagen in the dedifferentiated chondrocyte (control) are shown in FIGS. 2, 3 and 4, respectively. In FIGS. 2 to 4, BI denotes the medium containing BMP-2 and insulin, while BIT denotes the medium containing BMP-2, insulin and T3.

The results in FIGS. 2 to 4 show that the expression amount of type I collagen characteristic of the dedifferentiated chondrocyte decreases, the expression amount of type II collagen characteristic of the chondrocyte increases and the expression amount of type X collagen expressed when the cartilage is converted into the bone tends to be slightly increased as compared with the dedifferentiated chondrocyte as a control, when the dedifferentiated chondrocyte is cultured using the medium for redifferentiating the dedifferentiated chondrocyte into the chondrocyte in Example 1 (BI) containing insulin and BMP-2. The ratio (C/D) of the amount (C) of expression and production of type II collagen when the dedifferentiated chondrocyte is cultured using the medium in Example 1 (BI) for redifferentiating the dedifferentiated chondrocyte into the chondrocyte containing insulin and BMP-2 to the amount (D) of expression and production of type II collagen in the dedifferentiated chondrocyte as the control was about 10.

When the dedifferentiated chondrocyte is cultured using the medium for redifferentiating the dedifferentiated chondrocyte into the chondrocyte in Example 2 (BIT) containing insulin, BMP-2 and T3, on the other hand, the expression amount of type I collagen characteristic of the chondrocyte largely decreases, the expression amount of type II collagen characteristic of the dedifferentiated chondrocyte largely increases, and the expression amount of type X collagen expressed when the cartilage is converted into the bone tends to be slightly decreased as compared with the dedifferentiated chondrocyte as a control. The ratio (C/D) of the amount (C) of expression and production of type II collagen when the dedifferentiated chondrocyte is cultured using the medium for redifferentiating the dedifferentiated chondrocyte into the chondrocyte in Example 2 (BIT) containing insulin, BMP-2 and T3 to the amount (D) of expression and production of type II collagen in the dedifferentiated chondrocyte as a control was 20 or more.

These results show that the chondrocyte can be redifferentiated into the chondrocyte by culturing the chondrocyte differentiated to the dedifferentiated chondrocyte by dedifferentiation using the redifferentiation medium for redifferentiating the dedifferentiated chondrocyte to chondrocyte according to Examples 1 and 2. It was also found that, when the redifferentiation medium for redifferentiating the dedifferentiated chondrocyte into the chondrocyte in Example 2 is used, the dedifferentiated chondrocyte can be efficiently and readily redifferentiated into the chondrocyte as compared with using the medium for redifferentiating the dedifferentiated chondrocyte into the chondrocyte in Example 1.

### - Study of preferable amount of each component in the medium for making dedifferentiated chondrocyte to be redifferentiated into chondrocyte -

Preferable ranges of the contents of insulin, BMP-2 and T3 in the medium for redifferentiating the dedifferentiated chondrocyte into the chondrocyte in Example 2 containing insulin, BMP-2 and T3 were elucidated by the following three comparisons.

First, when the contents of BMP-2 were changed to 400 ng/mL, 200 ng/mL and 100 ng/mL, respectively, with a content of insulin of 5 µg/mL and a content of T3 of 10-7 M, the expression amount of type II collagen was the largest when the content of BMP-2 was 200 ng/mL (FIG. 5).

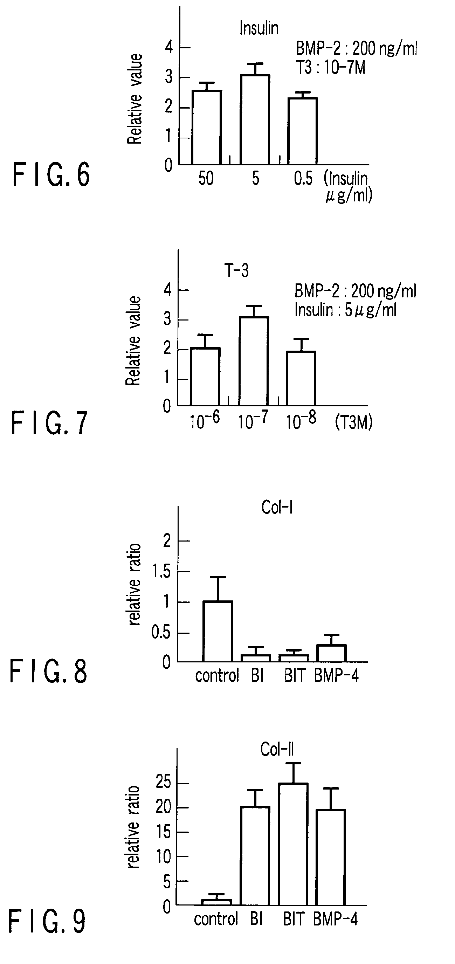

Second, when the contents of T3 were changed to 10-6 M, 10-7 M and 10-8 M, respectively, with a content of insulin of 5 µg/mL and a content of BMP-2 of 200 ng/mL, the expression amount of type II collagen was the largest when the content of T3 was 10-7 M (FIG. 6).

Third, the contents of insulin were changed to 50 µg/mL, 5 µg/mL and 0.5 µg/mL, respectively, with a content of BMP-2 of 200 ng/mL and a content of T3 of 10-7 M, the expression amount of type II collagen was the largest when the content of insulin was 5 µg/mL (FIG. 7).

The results above showed that the redifferentiating ability of the dedifferentiated chondrocyte into the chondrocyte was excellent when the contents of insulin, BMP-2 and T3 were 5 µg/mL, 200 ng/mL and 10-7 M, respectively, in the medium for redifferentiating the differentiated chondrocyte to chondrocyte in Example 2.

### - Effect of analogue -

The effect of the analogue on redifferentiation of the dedifferentiated chondrocyte into the chondrocyte was studied as follows by changing BMP-2 to its analogue BMP-4 in the medium of the invention for redifferentiating the dedifferentiated chondrocyte into the chondrocyte. The dedifferentiated chondrocyte was cultured as described previously using the same culture media for redifferentiating the dedifferentiated chondrocyte into the chondrocyte in Examples 1 and 2 and one in which BMP-2 was changed to BMP-4 in Example 2. The amounts of expression of type I collagen, type II collagen and type X collagen in the cultured dedifferentiated chondrocyte were measured. The amounts of expression of type I collagen, type II collagen and type X collagen in the dedifferentiated chondrocyte (control) are shown in FIGS. 8, 9 and 10, respectively. In FIGS. 8 to 10, "BI" denotes the medium containing BMP-2 and insulin; "BIT" denotes the medium containing BMP-2, insulin and T3; and "BMP-4" denotes the medium containing BMP-4, insulin and T3.

The ratio (C/D) of the amount (C) of expression and production of type II collagen obtained by culturing the dedifferentiated chondrocyte using the medium containing insulin, BMP-4 and T3 to the amount (D) of expression and production of type II collagen in the dedifferentiated chondrocyte (control) was 20 or more.

The results in FIGS. 8 to 10 show that the redifferentiation medium shows approximately the same redifferentiating ability as in Example 2 even when BMP-2 is changed to its analogue BMP-4 in the medium for redifferentiating the dedifferentiated chondrocyte to chondrocyte in Example 2.

### - Mechanical properties of the cultured product of redifferentiated chondrocyte -

The chondrocyte (P4) at fourth subculture about 30 days after the start of culture, or 200,000 cells of the chondrocyte differentiated to the dedifferentiated chondrocyte, were cultured by embedding in an aterocollagen three dimensional matrix for 21 days. The culture media used were the medium for redifferentiating the dedifferentiated chondrocyte into the chondrocyte used in Example 1, which contains 5 µg/mL of insulin (manufactured by MP Biomedicals Co.) and 200 ng/mL of BMP-2 (human recombinant bone morphogenetic protein-2, manufactured by Yamanouchi Pharmaceutical Co.) in a basal medium Dulbecco's Modified Eagle's Medium Nutrient Mixture F-12 HAM (trade name: DMEM/F12, manufactured by Sigma Chemical Co.), and the redifferentiation medium for redifferentiating the dedifferentiated chondrocyte into the chondrocyte used in Example 2, which contains 10-7 M of T3 (L-3,3',5'-triiodothyronine, manufactured by EMD Bioscience Co.) in addition to the above-mentioned components in the basal medium. These culture methods correspond to the method for redifferentiating the dedifferentiated chondrocyte into the chondrocyte according to the invention.

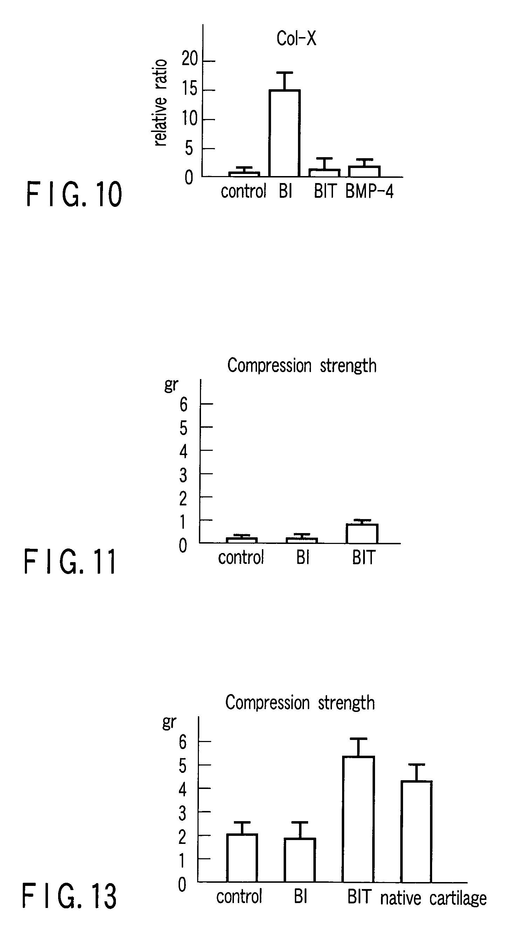

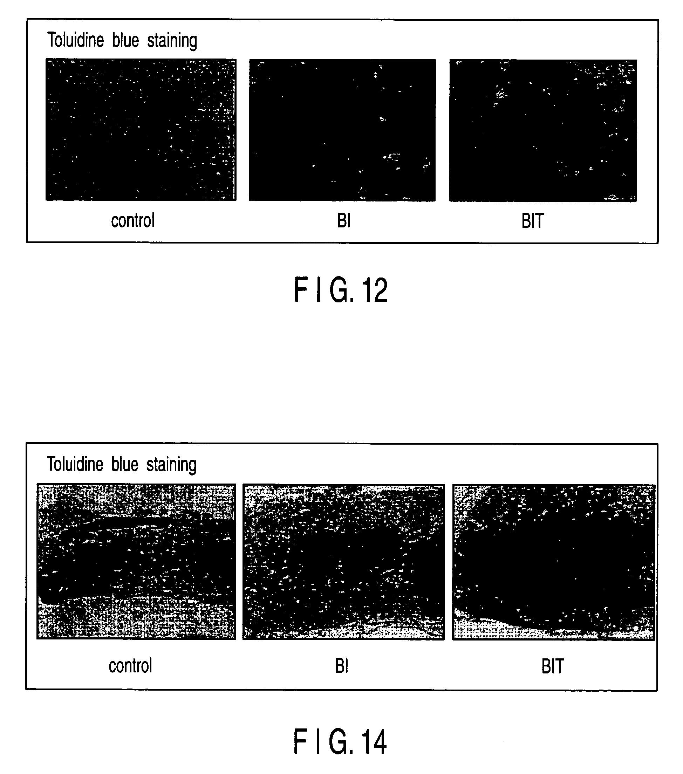

As shown in FIG. 11, the compression strength (gr) (measured with VENUSTRON, manufactured by Axsym Co.) as a mechanical property of the three-dimensional culture product by the three-dimensional culture showed an increasing tendency as compared with the culture product of chondrocyte (control) cultured in a medium containing no insulin, BMP-2 and T3 (see FIGS. 11 and 12). In FIGS. 11 and 12, "BI" denotes the medium containing BMP-2 and insulin, while "BIT" denotes the medium containing BMP-2, insulin and T3.

### - Mechanical properties of cultured product of the redifferentiated chondrocyte -

The dedifferentiated chondrocyte (200,000 cells) was cultured in the three-dimensional matrix of aterocollagen for 21 days using the medium for redifferentiating the dedifferentiated chondrocyte into the chondrocyte in Example 1 and the medium for redifferentiating the dedifferentiated chondrocyte into the chondrocyte in Example 2. The culture product was implanted to a node mouse for 2 months, and the compression strength (gr) of the transplanted culture product was measured as described above.

As shown in FIG. 13, the compression strength (gr) of the culture product, which was obtained by using the medium for redifferentiating the dedifferentiated chondrocyte into the chondrocyte according to Example 2, was higher than the culture product of the chondrocyte as a control, and the compression strength was approximately the same as the auricular cartilage (native cartilage) in the living body (see FIGS. 13 and 14). In FIGS. 13 and 14, "BI" denotes the medium containing BMP-2 and insulin, while "BIT" denotes the medium containing BMP-2, insulin and T3.

The medium for redifferentiating the dedifferentiated chondrocyte into the chondrocyte and the method for redifferentiating the dedifferentiated chondrocyte into the chondrocyte according to the invention are able to efficiently and readily redifferentiate the dedifferentiated chondrocyte, in which characteristics of the cartilage have been attenuated by dedifferentiation during in vitro culture, into the chondrocyte, and can be favorably used for continued culture. The chondrocyte cultured and proliferated by redifferentiation from the dedifferentiated chondrocyte using the medium for redifferentiating the dedifferentiated chondrocyte into the chondrocyte and the method for redifferentiating the dedifferentiated chondrocyte into the chondrocyte according to the invention may be favorably used as implantation materials for deficient or impaired portions of the cartilage of nose, ear, trachea and joint, and as cosmetic materials for the cosmetic surgery, and is quite useful for regenerative medicine of the cartilage.

## Claims

1. A redifferentiation medium used for making dedifferentiated chondrocyte to be redifferentiated into original chondrocyte, cartilage characteristics of said dedifferentiated chondrocyte having been attenuated due to dedifferentiation, said medium comprising: insulin; and at least one member selected from the group consisting of BMP-2 and analogues thereof.

2. The redifferentiation medium for making dedifferentiated chondrocyte to be redifferentiated into chondrocyte according to claim 1, further comprising T3.

3. The redifferentiation medium for making dedifferentiated chondrocyte to be redifferentiated into chondrocyte according to any one of claim 1 and 2, wherein the analogue of BMP-2 is BMP-4.

4. The redifferentiation medium for making dedifferentiated chondrocyte to be redifferentiated into chondrocyte according to any one of claims 1 to 3, wherein the insulin is present at a concentration from 0.05 to 500 µg/mL.

5. The redifferentiation medium for making dedifferentiated chondrocyte to be redifferentiated into chondrocyte according to any one of claims 1 to 4, wherein the at least one member selected form the group consisting of BMP-2 and analogues thereof is present at a concentration from 1 ng/mL to 40 µg/mL.

6. The redifferentiation medium for making dedifferentiated chondrocyte to be redifferentiated into chondrocyte according to any one of claims 1 to 5, wherein T3 is present at a concentration from 10-9 to 10-5 M.

7. The redifferentiation medium for making dedifferentiated chondrocyte to be redifferentiated into chondrocyte according to any one of claims 1 to 6, further comprising at least one member selected from the group consisting of fibroblast growth factor 2 (FGF-2), insulin-like growth factors (IGF-1), parathyroid hormone (PTH), growth hormone (GH), glucocorticoid, vitamin D, IL-1 receptor antagonist, estrogen, androgen, transformation growth factor α (TGFα), transformation growth factor β (TGFβ), bone morphogenic proteins (BMP), epidermal growth factor, platelet-derived growth factors, transferrin, selenious acid, linoleic acid, albumin, ascorbic acid, chondromodulins, heparin binding factor, α-fibroblast growth factor, vascular endothelial growth factor, mitogenic hormone, connective tissue growth factor, hepatocyte growth factor, arachidonic acid, prostaglandin A, prostaglandin B, prostaglandin E, prostaglandin F and histamine.

8. The redifferentiation medium for making dedifferentiated chondrocyte to be redifferentiated into chondrocyte according to any one of claims 1 to 7, wherein the redifferentiated chondrocyte is selected from hyaline chondrocyte and elastic chondrocyte.

9. The redifferentiation medium for making dedifferentiated chondrocyte to be redifferentiated into chondrocyte according to any one of claims 1 to 8, wherein the redifferentiated chondrocyte is cultured by any one of plate culture, three-dimensional culture and pellet culture.

10. A redifferentiation method for making dedifferentiated chondrocyte to be redifferentiated into original chondrocyte, cartilage characteristics of said dedifferentiated chondrocyte having been attenuated due to dedifferentiation, said method comprising the step of redifferentiating the dedifferentiated chondrocyte into the original chondrocyte by culturing the dedifferentiated chondrocyte using the medium according to any one of claims 1 to 9.

11. The redifferentiation method according to claim 10, wherein the culture is continued for 3 to 6 weeks.
